# EUROPÄISCHE PATENTANMELDUNG

(11) **EP 1 113 267 A2**
(43) Veröffentlichungstag der Anmeldung: **04.07.2001**
(21) Anmeldenummer: 00124842.6
(22) Anmeldetag: 14.11.2000
(51) Int. Cl.: G01N 33/00

(54) **Verfahren und Vorrichtung zur Messung von Gasinhaltsstoffen**

(30) Priorität: 03.12.1999 DE 19958441
(71) Anmelder: SIEMENS AKTIENGESELLSCHAFT, 80333 München (DE)
(72) Erfinder: Fischer, Bernhard, 84513 Töging a. Inn (DE); Gottlieb, Bernhard, Dr., 81739 München (DE); Kappel, Andreas, Dr., 85649 Brunnthal (DE); Kornely, Susanne, 80689 München (DE); Magori, Erhard, 85551 Kirchheim (DE); Meixner, Hans, Prof., 85540 Haar (DE); Mock, Randolf, Dr., 81379 München (DE); Unger, Martin, 82110 Germering (DE)

(57) **Zusammenfassung**

Die Erfindung betrifft ein Verfahren und eine Vorrichtung zur Messung von Gasinhaltsstoffen, wobei aus einem Hauptgasstrom über einen Bypass (1) ein Teilgasstrom abgezweigt wird, der nach dem Durchströmen einer Meßstrecke (5) wieder dem Hauptgasstrom zugeführt wird. Um auch bei einer Variation der Strömungsgeschwindigkeit in der Hauptgasströmung repräsentative Meßwerte zu erhalten, wird erfindungsgemäß vorgeschlagen, daß der Volumenstrom über die Meßstrecke (5) des Bypasses (1) unabhängig vom Volumenstrom im Hauptgasstrom konstant gehalten wird.

## Beschreibung

Die Erfindung betrifft ein Verfahren zur Messung von Gasinhaltsstoffen, insbesondere im Abgas einer Verbrennungskraftmaschine, nach dem Oberbegriff des Patentanspruchs 1. Weiterhin betrifft die Erfindung eine Vorrichtung zur Durchführung dieses Verfahrens.

Aus der Praxis ist es bekannt, zur Analyse der Schadstoffe im Abgas eines Kraftfahrzeugs in Strömungsrichtung der Abgase hinter dem Katalysator einen Bypass zum Abgasrohr und somit zur Hauptgasströmung anzuordnen. Diese bekannten Bypassschaltungen bestehen aus einem in der Hauptgasströmung angeordneten Einlauf zum Abzweigen eines Teilgasstroms, einer Meßstrecke mit darin angeordneter Sensorkammer sowie einem Rücklauf, über den der abgezweigte Teilgasstrom in den Hauptgasstrom im Abgasrohr zurückgeführt wird. Der Rücklauf kann dabei entweder vor oder hinter dem Endschalldämpfer in das Abgasrohr münden und weist in Strömungsrichtung des Hauptgasstroms, so daß am Rücklauf kein Staudruck anliegt.

Die als Teilgasstrom in den Bypass abgezweigte Gasmenge wird durch den am Einlauf abgegriffenen Staudruck bestimmt, wenn der Rücklauf vor dem Endschalldämpfer in das Abgasrohr mündet. Bei der Einmündung des Rücklaufs in das Abgasrohr hinter dem Endschalldämpfer wird die abgezweigte Gasmenge durch die Summe aus dem durch den Strömungswiderstand des Endschalldämpfers verursachten Druckabfall am Endschalldämpfer und dem Staudruck bestimmt. Der Staudruck am Einlauf wächst proportional zum Quadrat der Strömungsgeschwindigkeit im Abgasrohr, wobei die Strömungsgeschwindigkeit linear an die Drehzahl des Motors gekoppelt ist. Da sich der Volumenstrom für eine laminare Strömung im Bypass proportional zur Druckdifferenz zwischen Einlauf und Rücklauf ändert, die wiederum dem Staudruck entspricht, kann hieraus eine Schwankung des Volumenstroms im Bypass um den Faktor 36 zwischen Leerlauf und Vollast resultieren. Für eine quantitative Analyse, beispielsweise des NO-Gehalts im Abgas ist eine solche Schwankung aber nicht tolerierbar, weil das Sensorsignal von der Stärke des abgezweigten Teilgasstroms abhängt.

Davon ausgehend liegt der Erfindung die Aufgabe zugrunde, ein Verfahren zur Messung von Gasinhaltsstoffen zu schaffen, daß auch bei einer Variation der Strömungsgeschwindigkeit im Hauptgasstrom repräsentative Meßergebnisse liefert. Weiterhin liegt der Erfindung die Aufgabe zugrunde, eine Vorrichtung zur Durchführung des Verfahrens bereitzustellen.

Die Aufgabe wird verfahrensmäßig durch die im Patentanspruch 1 genannten Merkmale gelöst. Vorrichtungsmäßig wird die Aufgabenstellung durch die im Patentanspruch 3 genannten Merkmale gelöst.

Das erfindungsgemäße Verfahren sowie die erfindungsgemäße Vorrichtung haben den Vorteil, daß der Volumenstrom in der Meßstrecke des Bypass durch eine Druckregulierung konstant gehalten wird, wodurch erstmalig eine lastunabhängige Analyse der Gasinhaltsstoffe möglich ist.

Weitere Merkmale und Vorteile der Erfindung ergeben sich aus der nachfolgenden Beschreibung der zugehörigen Zeichnung, in der vier Ausführungsbeispiele einer erfindungsgemäßen Vorrichtung schematisch dargestellt sind. In der Zeichnung zeigt:
- Figur 1: ein Prinzipschaltbild einer ersten Schaltungsvariante einer erfindungsgemäßen Vorrichtung;
- Figur 2: ein Prinzipschaltbild einer zweiten Schaltungsvariante einer erfindungsgemäßen vorrichtung;
- Figur 3a: eine schematische Seitenansicht der in Figur 1 und 2 dargestellten ersten Ausführungsform eines Druckreglers;
- Figur 3b: eine entlang der Schnittlinie IIIb-IIIb gemäß Figur 3a geschnittene schematische Draufsicht;
- Figur 4: eine schematische Seitenansicht einer zweiten Ausführungsform eines Druckreglers und
- Figur 5: eine schematische Seitenansicht einer dritten Ausführungsform eines Druckreglers.

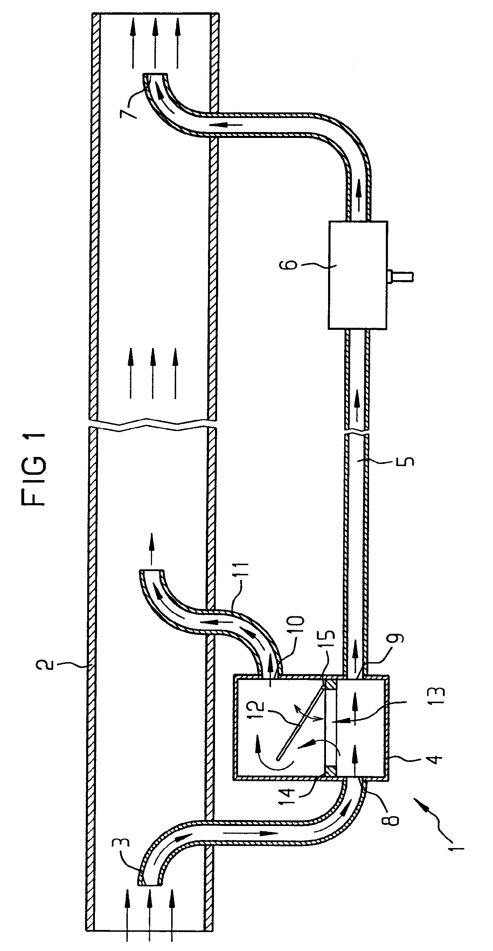

Das in Figur 1 dargestellte Prinzipschaltbild zeigt die Anordnung eines Bypasses 1 parallel zu einem Abgasrohr 2 eines Kraftfahrzeugs. Ein solcher Bypass 1 wird in Strömungsrichtung der Abgase hinter dem Katalysator eingesetzt, um einen Teilgasstrom aus dem Hauptgasstrom des Abgasrohres 2 zu Analysezwecken abzuzweigen. Eine solche Gasanalyse dient dazu, den Schadstoffgehalt, beispielsweise den NOₓ-Gehalt in dem Abgas zu bestimmen.

Der dargestellte Bypass 1 besteht aus einem im Abgasrohr 2 angeordneten Einlauf 3, über den aus dem Hauptgasstrom ein Teilgasstrom abgezweigt wird, einer nahe dem Einlauf 3 angeordneten Druckreguliereinheit, die im dargestellten Ausführungsbeispiel als mechanischer Druckregler 4 ausgebildet ist, einer Meßstrecke 5 mit darin angeordneter Meßkammer 6 sowie einem im Abgasrohr 2 einmündenden Rücklauf 7, über den der abgezweigte Teilgasstrom vor oder hinter dem Endschalldämpfer zurück in den Hauptgasstrom geleitet wird.

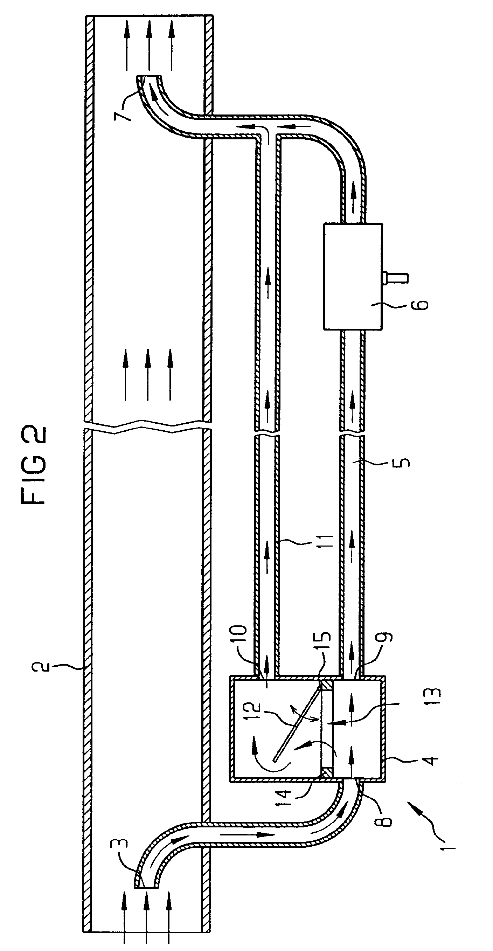

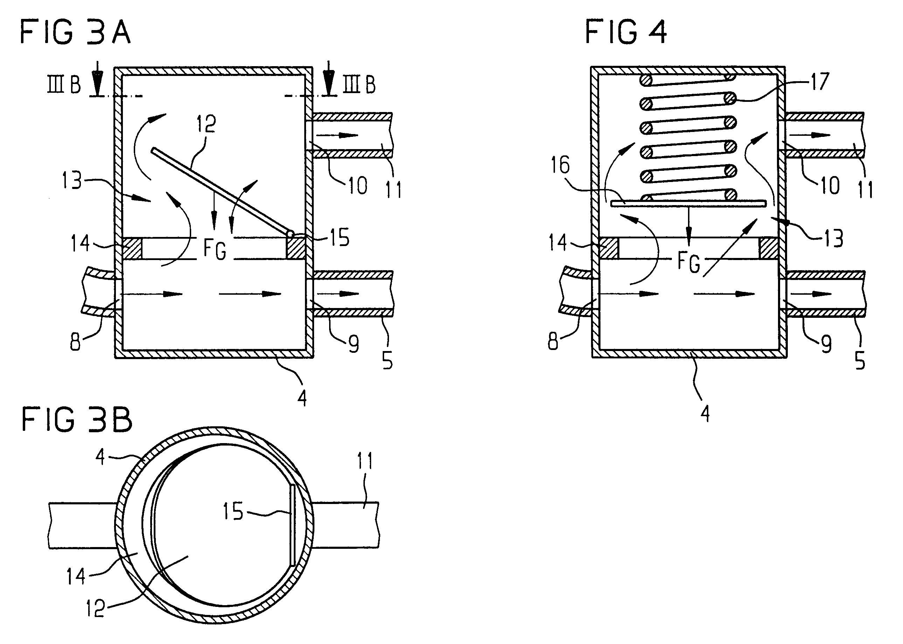

Der in den Prinzipschaltbildern gemäß Figur 1 und 2 dargestellte Druckregler 4 ist vergrößert in Figur 3a wiedergegeben. Der über den Einlauf 3 abgezweigte Teilgasstrom tritt über einen Zulauf 8 in den Druckregler 4 ein und kann diesen über die Abläufe 9 und 10 wieder verlassen, wobei der Ablauf 9 mit der Meßstrecke 5 verbunden ist und an den Ablauf 10 ein Überlauf 11 angeschlossen ist, über den ein Teil des Teilgasstroms an der Meßstrecke 5 vorbei wieder zurück in den Hauptgasstrom geleitet wird. Bei der Ausführungsform gemäß Figur 1 mündet der Überlauf 11 in ähnlicher Weise wie der Rücklauf 7 das Abgasrohr 2. Das in Figur 2 dargestellte Schaltungsprinzip unterscheidet sich von dem gemäß Figur 1 dadurch, daß der Überlauf 11 in Strömungsrichtung hinter der Meßstrecke 5 in den Bypass 1 mündet und der an der Meßstrecke 5 vorbeigeleitete Teilgasstrom über den Rücklauf 7 zurück in den Hauptgasstrom im Abgasrohr 2 gelangt.

Da das in der Meßkammer 6 gemessene Signal von der Stärke des abgezweigten und durch den Bypass 1 geleiteten Teilgasvolumenstrom abhängt, ist es für repräsentative Messungen notwendig, den Volumenstrom zumindest in der Meßstrecke 5 des Bypasses 1 konstant und somit unabhängig von der Strömungsgeschwindigkeit des Hauptgasstroms im Abgasrohr 2 zu halten.

Zu diesem Zweck ist im Bypass 1 nahe dem Einlauf 3 der Druckregler 4 angeordnet. Da der Volumenstrom durch den Bypass 1 proportional zur Druckdifferenz zwischen Einlauf 3 und Rücklauf 7 des Bypasses 1 ist, kann durch die Druckregulierung mittels des Druckreglers 4 für einen konstanten Volumenstrom in der Meßstrecke 5 gesorgt werden. Neben der in den Abbildungen dargestellten Ausführungsformen mechanischer Druckregler 4 ist es selbstverständlich auch möglich, auch nach anderen Prinzipien arbeitende Druckreguliereinheiten, wie beispielsweise elektronische Druckregler, zu verwenden.

Bei der in Figur 3a dargestellten ersten Ausführungsform eines mechanischen Druckreglers 4 weist der Druckregler 4 ein als einseitig verschwenkbar gelagerte Klappe 12 ausgebildetes Druckregelelement 13 auf. Durch das Druckregelelement 13, welches in der geschlossenen Stellung auf einer auf der Innenseite des Gehäuses des Druckreglers 4 angeordneten, umlaufenden Auflage 14 aufliegt, ist der den zum Überlauf 11 führenden Ablauf 10 aufweisende Gehäuseteil gegenüber dem den Zulauf 8 und den zur Meßstrecke 5 führenden Ablauf 9 aufweisenden Gehäuseteil verschließbar.

Die das Druckregelelement 13 bildende Klappe 12 ist um eine auf der Auflage 14 oder an der Innenseite des Gehäuses des Druckreglers 4 angeordnete Achse 15 verschwenkbar, wobei die Lagerung der Klappe 12 beispielsweise über ein Scharnier erfolgen kann.

Ein solchermaßen ausgestalteter Druckregler 4 arbeitet wie folgt:

Ist die Klappe 12 geschlossen, d.h. liegt sie auf der Auflage 14 auf, so fällt über die Klappe 12 der volle Staudruck ab. Durch diesen Druckabfall wird bezüglich der Achse 15 ein Drehmoment auf die Klappe 12 erzeugt, welches die Klappe 12 in Richtung der dargestellten Offenstellung verstellen will. Diesem durch den Druckabfall hervorgerufenen Drehmoment wirkt das Moment entgegen, das durch die Gewichtskraft FG der Klappe 12 erzeugt wird. Durch die Masse der Klappe 12 und/oder die Federkraft einer auf der Oberseite der Klappe 12 angeordneten - in Figur 3a nicht dargestellten - in Richtung der Kraft F_{G} wirkenden Feder läßt sich der Druck einstellen, ab dem sich die Klappe 12 öffnet.

Steigt beispielsweise aufgrund einer höheren Motordrehzahl der Staudruck aufgrund der durch die Drehzahlerhöhung hervorgerufenen erhöhten Strömungsgeschwindigkeit des Hauptgasstroms im Abgasrohr 2 über den eingestellten Druck an, so wird die Klappe 12 um die Achse 15 in die Offenstellung verschwenkt, wodurch der Zugang zum Ablauf 10 freigegeben wird und ein Teilvolumenstrom über den Überlauf 11 an der Meßstrecke 5 vorbei zurück in das Abgasrohr 2 geführt wird.

Sobald der Druckabfall über die Klappe 12 wieder im Bereich des voreingestellten Drucks liegt, schließt sich die Klappe 12 wieder selbsttätig. Durch dieses druckabhängige Öffnen und Schließen des als Klappe 12 ausgebildeten Druckregelelements 13 wird sichergestellt, daß unabhängig vom Staudruck über die Meßstrecke 5 immer der vorgegebene Druck abfällt und somit ein konstanter Volumenstrom die Meßkammer 6 durchströmt.

Der Aufbau von Klappe 12, Schwenkachse 15 und Auflagel4 für die Klappe 12 in der Schließstellung ist besonders gut der Abbildung Figur 3b zu entnehmen.

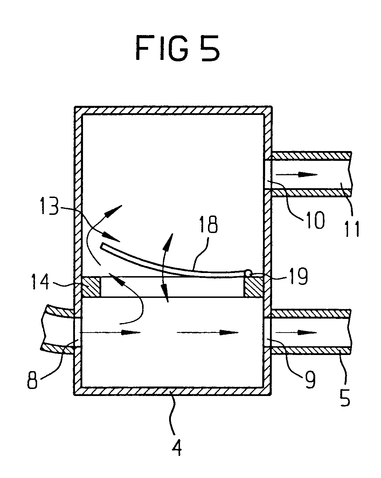

Figur 4 zeigt eine zweite Ausführungsform zur Ausgestaltung des Druckregelelements 13. Bei dieser Ausführungsform ist das Druckregelelement 13 als lose Platte 16 ausgebildet, die in der Schließstellung auf der auf der Innenseite des Gehäuses des Druckreglers 4 angeordneten, umlaufenden Auflage 14 aufliegt.

Auch bei dieser Ausführungsform des Druckregelelements 13 ist die Schließkraft über die Masse der Platte 16 und somit die Gewichtskraft FG einstellbar, die dem Öffnen der Platte 16 entgegenwirkt. Zusätzlich kann zum Einstellen des Drucks,ab dem sich die Platte 16 öffnen soll, auf der Oberseite der Platte 16 eine Feder 17 angeordnet sein, wie dies in Figur 4 dargestellt ist. Die Federkraft dieser beispielsweise als Wendelfeder Ausgebildeten Feder 17 wirkt in Richtung der Kraft F_{G} und somit in Richtung der Schließstellung der Platte 16.

Steigt der Druck am Zulauf 8 über den vorgegebenen Druck an, so wird die Platte 16 nach oben gedrückt, wodurch der Weg zum Überlauf 11 freigegeben wird, über den ein Teilvolumenstrom an der Meßstrecke 5 vorbei zum Hauptgasstrom im Abgasrohr 2 geführt wird. Sobald der Druckabfall über die Platte 16 wieder auf den vorgegebenen Wert absinkt, schließt sich die Platte 16 wieder selbsttätig. Auch bei dieser Ausführungsform wird sichergestellt, daß der Druck, der über die Meßstrecke 5 abfällt, unabhängig vom Staudruck im Abgasrohr 2 ist, was wiederum einen konstanten Volumenstrom durch die Meßkammer 6 gewährleistet.

Figur 5 zeigt schließlich eine dritte Ausführungsform eines Druckreglers 4, bei der das Druckregelelement 13 als einseitig festgelegte, vorgespannte Biegefeder 18 ausgebildet ist, die in der Schließstellung auf einer auf der Innenseite des Gehäuses des Druckreglers 4 angeordneten, umlaufenden Auflage 14 aufliegt. Die Schließkraft dieses als Biegefeder 18 ausgebildeten Druckregelelements 13 läßt sich über die Vorspannung und/oder die Materialeigenschaften der Biegefeder 18 einstellen.

Wenn der Druck am Zulauf 8 über den eingestellten Wert ansteigt, biegt sich die Biegefeder 18 um ein Einspannungl9 aufwärts, bis sie den Zugang zum Überlauf 11 freigibt und ein Teilvolumenstrom an der Meßstrecke 5 vorbei in das Abgasrohr 2 zurückgeführt wird, bis der Druckabfall über die Biegefeder 18 wieder unter den vorgegebenen Wert fällt. Auch diese dritte Variante zur Ausgestaltung des Druckreglers 4 stellt sicher, daß der Druckabfall über die Meßstrecke nicht vom Staudruck im Abgasrohr 2 abhängig ist und die Meßkammer 6 ein konstanter Volumenstrom durchströmt.

## Patentansprüche

1. Verfahren zur Messung von Gasinhaltsstoffen, insbesondere im Abgas einer Verbrennungskraftmaschine, wobei aus einem Hauptgasstrom über einen Bypass (1) ein Teilgasstrom abgezweigt wird, der nach dem Durchströmen einer Meßstrecke (5) wieder in den Hauptgasstrom zurückgeführt wird,
**dadurch gekennzeichnet**, daß der Volumenstrom des Teilgasstroms über die Meßstrecke (5) des Bypasses (1) unabhängig vom Volumenstrom im Hauptgasstrom konstant gehalten wird.

2. Verfahren nach Anspruch 1,
**dadurch gekennzeichnet**, daß die Druckdifferenz entlang der Meßstrecke (5) des Bypasses (1) konstant gehalten wird.

3. Vorrichtung zum Messen der Inhaltsstoffe eines Gases, insbesondere eines Abgasstroms einer Verbrennungskraftmaschine, insbesondere nach dem Verfahren nach Anspruch 1 oder 2, mit einem einen Einlauf (3), eine Meßstrecke (5) und einen Rücklauf (7) aufweisenden Bypass (1), über den ein aus dem Hauptgasstrom abgezweigter Teilgasstrom der Meßstrecke (5) zuführbar ist,
**dadurch gekennzeichnet**, daß in dem Bypass (1) in Strömungsrichtung vor der Meßstrecke (5) eine Druckreguliereinheit angeordnet ist.

4. Vorrichtung nach Anspruch 3, **dadurch gekennzeichnet**, daß die Druckreguliereinheit einen Überlauf (11) aufweist, über den ein Teil des Teilgasstroms vor der Meßstrecke (5) abzweigbar ist.

5. Vorrichtung nach Anspruch 4,
**dadurch gekennzeichnet**, daß der Überlauf (11) in den Hauptgasstrom mündet.

6. Vorrichtung nach Anspruch 4,
**dadurch gekennzeichnet**, daß der Überlauf (11) in Strömungsrichtung hinter der Meßstrecke (5) in den Bypass (1) mündet.

7. Vorrichtung nach mindestens einem der Ansprüche 3 bis 6, **dadurch gekennzeichnet**, daß die Druckreguliereinheit nahe dem Einlauf (3) im Bypass (1) angeordnet ist.

8. Vorrichtung nach mindestens einem der Ansprüche 3 bis 7, **dadurch gekennzeichnet**, daß die Druckreguliereinheit als mechanischer Druckregler (4) ausgebildet ist.

9. Vorrichtung nach Anspruch 8
**dadurch gekennzeichnet**, daß der Druckregler (4) ein Gehäuse aufweist, in den der durch den Bypass (1) abgezweigte Teilgasstrom über einen Zulauf (8) einströmen und über voneinander getrennte Abläufe (9, 10) zur Meßstrecke (5) und zum Überlauf (11) ausströmen kann, wobei der den Ablauf (10) zum Überlauf (11) aufweisende Gehäuseteil über ein Druckregelelement (13) gegenüber dem den Zulauf (8) und den Ablauf (9) zur Meßstrecke (5) aufweisenden Gehäuseteil verschließbar ist.

10. Vorrichtung nach Anspruch 9,
**dadurch gekennzeichnet**, daß das Druckregelelement (13) als einseitig gelagerte, verschwenkbare Klappe (12) ausgebildet ist, die in der Schließstellung auf einer auf der Innenseite des Gehäuses angeordneten, umlaufenden Auflage (14) aufliegt.

11. Vorrichtung nach Anspruch 9,
**dadurch gekennzeichnet**, daß das Druckregelelement (13) als in der Schließstellung auf einer auf der Innenseite des Gehäuses angeordneten, umlaufenden Auflage (14) lose aufliegende Platte (16) ausgebildet ist.

12. Vorrichtung nach Anspruch 10 oder 11
**dadurch gekennzeichnet**, daß das Druckregelelement (13) aufgrund der Schwerkraft und/oder Federkraft bis zu einem vorgebbaren Druck in der Schließstellung haltbar ist.

13. Vorrichtung nach Anspruch 9,
**dadurch gekennzeichnet**, daß das Druckregelelement (13) als einseitig festgelegte, vorgespannte Biegefeder (18) ausgebildet ist, die in der Schließstellung auf einer auf der Innenseite des Gehäuses angeordneten, umlaufenden Auflage (14) aufliegt.

14. Vorrichtung nach Anspruch 13,
**dadurch gekennzeichnet**, daß die Schließkraft der Biegefeder (18) über die Vorspannung und/oder die Materialeigenschaft der Biegefeder (18) einstellbar ist.
